(19) Europäisches Patentamt

European Patent Office

Office européen des brevets

(11) **EP 1 402 861 A1**

(12) **EUROPEAN PATENT APPLICATION**

(43) Date of publication:
**31.03.2004 Bulletin 2004/14**

(51) Int Cl.[7]: **A61F 13/15**, A61L 15/22,
A61L 15/60

(21) Application number: **02021369.0**

(22) Date of filing: **24.09.2002**

(84) Designated Contracting States:
**AT BE BG CH CY CZ DE DK EE ES FI FR GB GR
IE IT LI LU MC NL PT SE SK TR**
Designated Extension States:
**AL LT LV MK RO SI**

(71) Applicant: **The Procter & Gamble Company**
**Cincinnati, Ohio 45202 (US)**

(72) Inventors:
• **Gagliardi, Ivano**
  **65123 Pescara (IT)**

• **D'Addario, Roberto**
  **65019 Pianella (IT)**
• **Carlucci, Giovanni**
  **66100 Chieti (IT)**
• **Veglio, Paolo**
  **65129 Pescara (IT)**

(74) Representative: **Kremer, Véronique et al**
  **Procter & Gamble Service GmbH**
  **65823 Schwalbach am Taunus (DE)**

(54) **Flexible disposable absorbent articles**

(57) The present invention relates to an absorbent article, namely an absorbent article for feminine protection, having the property of being more flexible in use condition, thereby providing improved comfort to the wearer of the article, than before use, i.e., upon handling and positioning of the article on the undergarment of the wearer.

Figure 1

**Description**

**Field of the invention**

The present invention relates to disposable absorbent articles, namely absorbent articles for feminine protection such as sanitary napkins, panty liners, and incontinence pads. More particularly the present invention relates to disposable absorbent articles having the property of being more flexible in use conditions, thereby providing improved comfort to the wearer of the articles, than before use, i.e., upon handling of the articles per the wearer and positioning on the undergarment of the wearer.

**Background of the invention**

Absorbent articles suitable for feminine protection such as sanitary napkins, panty liners, and incontinence pads are typically worn in the crotch region of an undergarment and attached to the undergarment by a so called panty-fastening-adhesive. In order to be comfortable to the wearer these articles need to be flexible. It is believed that the more flexible an absorbent article is, the less will it be noticeable to the wearer. Hence this provides comfort by more closely resembling the situation when no such absorbent article is worn.

Flexibility can easily be achieved by reducing the amount of material in an absorbent article or replacing stiff/inflexible components by more flexible ones. However such routes might not be satisfactory as such changes may typically affect the overall performance of the article like protection. It has now been recognized that extreme flexibility although desirable in use conditions is somehow less desirable before use conditions. Indeed a too flexible article may be difficult to handle for the wearer before use typically when removing the release liner and attaching it to the undergarment.

There is hence still the unsatisfied consumer need for an absorbent article offering enhanced fit and comfort through a construction which promotes a continuously self-conforming anatomical cooperation of the article to the wearer to yield a highly effective absorbent article. Indeed, there is a need for absorbent article suitable for feminine protection with high flexibility properties during use while still maintaining close contact to the puddental region even upon movement of the wearer's legs, thereby exhibiting improved stay in place preferably while not compromising on other properties of the article. In other words, overall improved comfort is desired.

It is thus an object of the present invention to provide absorbent articles, namely absorbent articles for feminine protection, providing high comfort in use while being easy to handle per wearer before use. It is a further object of the present invention to provide these properties while not compromising on overall protection or even improving the overall protection (namely liquid absorption capacity) and hence improving overall comfort during usage experimentation.

It has now been found that these objects are met by providing an absorbent article having a flexibility at 38°C when measured in either longitudinal or transverse direction per stiffness test method as described herein after that is of 20% more than the flexibility of said article at 23°C when measured in same direction. In other words, the article according to the present invention is more flexible in use condition than before use condition, i.e., at room temperature before placement of the article in close proximity to the puddental region of a woman wearing the article. The article of the present invention is able to closely conform to the various anatomical shapes of the female urogenital and buttocks region, including upon movement of the legs of the wearer without the drawback of mechanical bunching, resulting thereby in immediate and direct take off of body fluid discharge per the article, thereby reducing soiling. Indeed, better fit and overall comfort is achieved due to the property of the article to become more flexible as the temperature increases, i.e. upon contact with puddental regions.

The present invention provides an absorbent article which has high flexibility properties in use condition and which is still stiff enough during handling per the wearer before it is worn in contact with the puddental region of the wearer. Advantageously the present invention overcomes these somehow conflicting technical requirements (high flexibility in use and relative stiffness before use), this especially in instance where ultra thin feminine protection articles are desired like panty liners.

In a preferred embodiment the absorbent articles of the present invention satisfy the need mentioned herein before while not compromising on the absorption performance of said article. Hence the preferred absorbent articles according to the present invention have a total liquid absorption capacity of at least 1 gram as measured on the entire article per the Dunk absorption capacity test described herein.

**Background art**

Flexible absorbent articles are already known in the art. For example EP-A-705 586, EP-A-705 584 or EP-A-336 578. These references only give range of suitable flexibility for the articles. However none of these references discloses, nor suggests article having the ability to be more flexible in use condition, i.e. once worn in contact to puddental region of wearer, than before use, e.g., upon handling of the article per the wearer before placement in the undergarment.

Separately liquid absorbent thermoplastic compositions comprising a thermoplastic polymeric base material and absorbent material as well as their use to bond different layers, for example absorbent core to the topsheet or backsheet, of conventional disposable absorbent articles such as diapers or sanitary napkins are disclosed in for example EP-A-101329, WO99/57201 and WO02/07791.

### Summary of the invention

The present invention encompasses an absorbent article typically for feminine protection, particularly suitable for use in an undergarment. The article has a flexibility in longitudinal or transverse direction when measured according to stiffness tester AB Lorentzen & Wettre at 38°C that is of at least 20% more than the flexibility of said article when measured at 23°C in same direction.

All documents cited are, in relevant part, incorporated herein by reference; the citation of any document is not to be construed as an admission that it is prior art with respect to the present invention.

### Brief description of the drawings

Figure 1 shows a plan view of the garment facing surface of a panty liner according to the present invention.

Figure 2 shows a cross section view of the liner of Figure 1 taken through line D-D'.

Figure 3 shows the arrangement used to carry out the Dunk absorption capacity test

Figure 4 shows the stiffness tester (AB Lorentzen & Wettre) measuring flexibility of a sample by folding it for a certain angle, the cell measures the resistance of the sample to the folding.

### Detailed description of the invention

The term "absorbent article" is used herein in a very broad sense including any article able to receive and/or absorb and/or contain and/or retain fluids and/or exudates, especially body fluids/body exudates. "Absorbent articles" as referred to herein include, without to be limited to, sanitary napkins, panty liners, incontinence pads, interlabial pads, breast pads and the like.

The term "disposable" is used herein to describe articles that are not intended to be launched or otherwise restored or reused as an article (i.e., they are intended to be discarded after a single use and, preferably to be recycled, composted or otherwise disposed of in an environmentally compatible manner).

As used herein, the term 'wearer-facing' surface refers to the surface of the component of the article generally oriented to face the wearer skin and/or mucosal surface during use of the article. As used herein, the term 'garment facing' surface refers to the opposite outer surface of the article, typically the surface directly facing the garment of a wearer, if worn in direct contact with garment.

As used herein, the term 'body fluids and/or body exudates' refers to any fluids/exudates produced by human body occurring naturally or accidentally like for instance in the case of skin cutting, including for instance perspiration, urine, menstrual fluids, faeces, vaginal secretions and the like.

The absorbent article according to the present invention typically comprises a liquid pervious surface in order to allow liquid to pass into the article, a liquid impervious surface providing liquid containment such that absorbed liquid does not leak through the article and an absorbent element comprised there between providing the absorbent capacity of the article to acquire and retain liquid which has entered through the liquid pervious surface. Usually, such as in the absorbent articles described herein as examples of the present invention, the liquid pervious surface is provided by a topsheet and the liquid impervious surface is provided by a backsheet.

In order to realize the benefits of the present invention the absorbent article as a whole needs to provide different flexibility properties at different temperatures, namely before use of the article at room temperature and during use of the article in close proximity of the puddental region of a wearer at body temperature.

As an essential feature the absorbent articles according to the present invention have a flexibility when measured either in longitudinal and/or transversal direction at 38°C that is of at least 20% more than the flexibility of said article measured at 23°C in same direction. It is to be understood herein that flexibility differential requirement at the two different temperatures as per detailed test method described herein after is met if at least this differential is obtained in one of the two directions, i.e. either longitudinal (machine direction - MD) or transverse (cross direction - CD). In a preferred embodiment herein this differential is obtained in both CD and MD direction.

Preferably the absorbent article according to the present invention has a flexibility when measured either in longitudinal and/or transversal direction at 38°C that is of 25% more than the flexibility in same direction of said article when measured at 23°C, preferably 30% more and most preferably from 32% more to 50% more.

The flexibility is measured by stiffness test method described herein after in longitudinal direction and/or transversal direction by using a commercially available stiffness tester, namely AB Lorentzen & Wettre, Stockholm, Sweden under reference app. 16 type 10-1 n 734 as described herein below.

The expression "flexibility" is also referred to as "drapability". It is understood herein that an increase in flexibility of a given percent when comparing a same article at two different temperatures, namely 23°C and 38°C, corresponds to a reduction of rigidity of the same percent when comparing the same article at said two temperatures. It should be understood that rigidity (also called stiffness) is characteristic of the opposite behavior of a material.

Typically the absorbent articles according to the present invention have a flexibility in longitudinal direction of 100 mN to 10 mN when measured according to AS Lorentzen & Wettre stiffness tester at 23°C as described herein after, preferably in the range of 80 to 30 mN and more preferably of 60 to 40 mN.

Typically the absorbent articles according to the present invention have a flexibility in longitudinal direction of 100 mN to 10 mN when measured with a AB Lorentzen & Wettre stiffness tester at 38°C as described herein after, preferably in the range of 70 to 15 mN and more preferably of 45 to 20 mN.

Typically the absorbent articles according to the present invention have a flexibility in transverse direction of 100 mN to 10 mN when measured with a AB Lorentzen & Wettre stiffness tester at 23°C as described herein after, preferably in the range of 80 to 30 mN and more preferably of 60 to 40 mN.

Typically the absorbent articles according to the present invention have a flexibility in transverse direction of 100 mN to 10 mN when measured with a AS Lorentzen & Wettre stiffness tester at 38°C as described herein after, preferably in the range of 70 to 15 mN and more preferably of 45 to 20 mN.

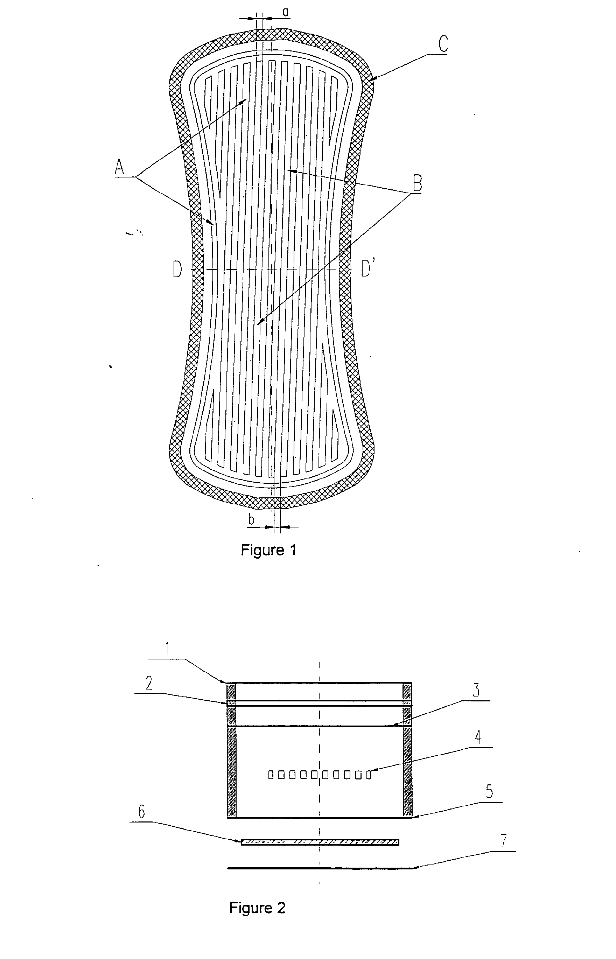

In a preferred embodiment herein the article has a flexibility in transverse direction which is at least 10% more than its flexibility in longitudinal direction when measured with a AS Lorentzen & Wettre apparatus as described herein after at 23°C, preferably at least 20% more, more preferably at least 40% more and most preferably at least 60% more. In highly preferred execution of the invention wherein the absorbent article is provided with an absorbent element comprising a liquid absorbent thermoplastic composition as described herein after configured in unattached spaced apart zones, namely stripes (as illustrated in for example Figures 1 and 2), the flexibility in transverse direction is 70% more than the flexibility in longitudinal direction when measured as described herein at 23°C. The flexibility in transverse direction (cross direction) is very important since it is the flexibility that wearer perceives more due to the contact between the article and the legs in the crotch area. Absorbent element configured in stripe as illustrated in Figure 1 reduces significantly the stiffness in cross direction matching this requirement.

This increased flexibility properties in correlation with increased temperature provides exceptional wearer comfort without soiling and/or absorbent performance problems due to bunching or densification of the absorbent material and still allows the wearer to attach the article to the undergarment without undue effort.

The degree of flexibility is determined by the selection of the materials for the components of the article and their respective quantity. Those skilled in the art will readily be able to identify materials which can be used in the context of the absorbent articles according to the present invention. Many absorbing articles and constructions, including particular materials, are known in the art and have been described in ample detail over time. All of such materials are useful in the context of the present invention, provided that they allow in combination in an article the flexibility behavior as claimed to be achieved. A main component of the article which heavily influences its flexibility behavior is its absorbent element. Therefore the following description of typical materials of the main components of the absorbent article will allow to provide an almost infinite number of article variants inside and outside the flexibility limitations according to the present invention. Whether or not an absorbent article meets the requirements of flexibility of the present invention can then be analyzed by simple measurements according to the method described herein.

It will be apparent to those skilled in the art that, in order to achieve the flexibility behavior according to the present invention, the selection of kind and quantity of raw materials has to be balanced with other desired characteristics of the absorbent article such as for example absorbent capacity, absorption speed and surface dryness on the outside of the topsheet during use.

In a preferred embodiment herein the absorbent element is such as the total liquid absorption capacity of the entire article is of at least 1 g, preferably from 2 to 80 g and most preferably from 4 to 40g, when measured per the Dunk absorption capacity test described herein after. If the absorbent article is a pantiliner its total liquid absorption capacity is most preferably from 4 g to 15g and if the absorbent article is a pad its total liquid absorption capacity is most preferably from 5 to 40g.

One preferred absorbent article of the present invention comprises an absorbent element comprising a liquid absorbent thermoplastic composition which comprises a thermoplastic polymeric base material having particles of water-insoluble water swellable absorbent material dispersed therein.

The liquid absorbent thermoplastic composition might be used for totally or partially substituting conventional fibrous absorbent element/core. The use of such liquid absorbent thermoplastic composition as element of the absorbent element in absorbent articles allows to provide the particular flexibility behavior mentioned herein while still providing outstanding liquid absorption capacity.

In the following, non-limiting embodiments of the main elements of the absorbent article are described.

*Absorbent element*

The absorbent element of the article according to the present invention typically includes the following components: (a) optionally one or more fluid distribution layer, (b) a fluid storage element and (c) optionally a fibrous layer underlying the storage element.

One optional component of the absorbent element according to the present invention is a fluid distribution layer. This distribution layer typically underlies the topsheet and is in fluid communication therewith. The topsheet transfers the acquired menstrual fluid to this primary distribution layer for ultimate distribution to the storage element. This transfer of fluid through the distribution layer occurs not only in the thickness, but also along the length and width directions of the absorbent article. Optionally there can be not only one fluid distribution layer but several fluid distribution layers. The purpose of any additional distribution layer is to readily acquire menstrual fluid from the primary fluid distribution layer and transfer it rapidly to the underlying storage element. This helps the fluid capacity of the underlying storage element to be fully utilized.

Positioned in fluid communication with, and typically underlying the topsheet, or optional primary or secondary fluid distribution layers if present, is a fluid storage element.

The fluid storage element is the essential element of the absorbent element and is able to ultimately acquire and retain body fluid. The storage element preferably comprises water-insoluble water swellable absorbent materials, also referred to as absorbent gelling materials.

The fluid storage element can comprise solely absorbent gelling materials, or these absorbent gelling materials can be dispersed homogeneously or non-homogeneously in a suitable carrier or it can comprise solely an absorbent carrier material.

Suitable carriers include cellulose fibers, in the form of fluff, tissues or paper such as is conventionally utilized in absorbent elements like cores. Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like.

Highly preferred carrier for use herein is a thermoplastic polymeric base material. Indeed, in a preferred embodiment herein the absorbent element, namely the storage element comprises a liquid absorbent thermoplastic composition comprising a polymeric base material having particles of water-insoluble water swellable absorbent material dispersed therein. Such absorbent element provides more than 50% of the total absorption capacity of the article, preferably more than 80%.

In a preferred embodiment herein the liquid absorbent thermoplastic composition represents at least 1% by weight of the total weight of the absorbent article, preferably from 5% to 90%, more preferably from 10% to 70%, even more preferably 15% to 50% and most preferably from 20% to 40%.

Typically the absorbent element comprises the liquid absorbent thermoplastic composition described herein at a percent by weight of the total weight of the absorbent element of at least 15%, preferably from 20% to 100%, more preferably from 30% to 90%, even more preferably from 45% to 85% and most preferably from 55% to 80%.

Water-insoluble but water-swellable absorbent materials or absorbent gelling materials are usually referred to as "hydrogels", "superabsorbent", "absorbent gelling" materials. Absorbent gelling materials are those materials that, upon contact with aqueous fluids, especially aqueous body fluids, imbibes such fluids and thus form hydrogels. These absorbent gelling materials are typically capable of absorbing large quantities of aqueous body fluids, and are further capable of retaining such absorbed fluids under moderate pressures. These absorbent gelling materials are typically in the form of discrete, non fibrous particles, even if superabsorbent in fiber form are known.

Any commercially available superabsorbent material in particle form is suitable for the present invention. Suitable absorbent gelling materials for use herein will most often comprise a substantially water-insoluble, slightly crosslinked, partially or fully neutralized, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Patent 4,654,039 and reissued as RE 32,649. Preferred monomers include acrylic acid, methacrylic acid, and 2-acrylamido-2-methyl propane sulfonic acid. Acrylic acid itself is especially preferred for preparation of the polymeric gelling material. The polymeric component formed from the unsaturated, acid-containing monomers can be grafted onto other types of polymer moieties such as starch or cellulose. Polyacrylate grafted starch materials of this type are especially preferred. Preferred polymeric absorbent gelling materials that can be prepared from conventional types of monomers include hydrolyzed acrylonitrile grafted starch, polyacrylate grafted starch, polyacrylates, maleic anhydride-based copolymers and combinations thereof. Especially preferred are the polyacrylates and polyacrylate grafted starch, but other absorbent substances in particle form such as inorganic materials ($MgSO_4$, $CaCl_2$, silicas, zeolites, etc.) or absorbing polymers (i.e.chitin derivatives such as, for example, chitosan) can be used alone but, preferably, blended with the above superabsorbent polymers.

In one embodiment herein the average particle size of the water insoluble water swellable material in dry state is below 150 micrometers, more preferably below 50 micrometers and most preferably from 40 to 10 micrometers. "Particle size" as used herein means the weighted average of the smallest dimension of the individual particles. Without to be bound by theory the smaller the particle size, the higher the liquid absorption capacity. Also small particles contribute to thin absorbent element. The minimun thickness of a layer of the absorbent element herein is determined by the particle size (diameter). Preferred water insoluble water swellable material for use herein have a substantially angle-lacking shape (i.e., are free of sharp angle on their surface) and preferably have a spherical shape. A spherical particle typically has a ratio of its largest cross dimension to its smallest cross dimension of less than 1.5 and preferably about 1. Suitable commercially available angle lacking absorbent gelling material is for example Aquakeep® 10SH-NF, available from Sumitomo Seika, having an average particle size of between 20 μm and 30 μm.

It is preferable hat the water insoluble water swellable material is present in an amount from 1% to 95%, preferably from 10% to 90% and most preferably 20% to 60% by weight of the liquid absorbent thermoplastic composition.

The liquid absorbent thermoplastic composition for use herein comprises as a further essential element a polymeric base material typically at a level from 5% to 99% per weight, preferably from 10% to 90%, more preferably from 30% to 70% and most preferably from 40% to 60% by weight of liquid absorbent thermoplastic composition. Any polymeric base material known to the skilled person and used in the construction of absorbent articles, such as feminine care absorbent articles (e.g. sanitary napkins, panty liners or incontinence articles) or baby care absorbent articles (e. g. diapers) can be used herein.

The polymeric base materials for use herein comprise thermoplastic polymers as an essential element. Thermoplastic polymer or mixtures of polymers are present in amounts typically ranging from about 5 wt% to about 99 wt %, preferably from about 10 wt % to about 90 wt % and most preferably from 40% to 60%, with respect to the total weight of the thermoplastic polymeric base material.

A variety of different thermoplastic polymers are suitable for use herein. Exemplary thermoplastic polymers include but are not limited to block copolymers, amorphous and crystalline polyolefins including homogeneous and substantially linear ethylene/alpha-olefin interpolymers, interpolymers of ethylene such as ethylene-vinyl-acetate (EVA), ethylene-methyl-acrylate (EMA) and ethylene n-butyl acrylate (EnBa) and mixtures thereof. The group of block copolymers includes linear copolymers of the triblock A-B-A or the diblock A-B type, or radial co-polymer structures having the formula $(A-B)_x$. The A blocks are non-elastic polymer blocks, typically polyvinylarene blocks, the B blocks are unsaturated conjugated dienes, such as poly(monoalkenyl) blocks, or hydrogenated versions thereof, x denotes the number of polymeric arms, and x is an integer greater than or equal to one. Suitable block A polyvinylarenes include, but are not limited to polystyrene, polyalpha-methylstyrene, polyvinyltoluene, and combinations thereof. Suitable block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include Europrene[TM] Sol T from EniChem, Kraton[TM] elastomers from Shell Chemical Company, Vector[TM] elastomers from Dexco, Solprene[TM] from Enichem Elastomers and Stereon[TM] from Firestone Tire & Rubber Co.

Amorphous polyolefins or amorphous polyalphaolefins (APAO) are homopolymers, copolymers, and terpolymers of $C_2$-$C_8$ alphaolefins. These materials are typically polymerised by means of processes, which employ Ziegler-Natta catalysts resulting in a relatively broad molecular weight distribution. Commercially available amorphous polyalphaolefins include Rextac[TM] and REXFlex[TM] propylene based homopolymers, ethylene-propylene copolymers and butene-propylene copolymers available from Rexene (Dallas, TX) as well as Vestoplast alpha-olefin copolymers available from Huls (Piscataway, NJ).

Metallocene polyolefins are homogeneous linear and substantially linear ethylene polymers prepared using single-site or metallocene catalysts. Homogeneous ethylene polymers are characterized as having a narrow molecular weight distribution and a uniform short-chain branching distribution. In the case of substantially linear ethylene polymers, such homogeneous ethylene polymers are further characterized as having long chain branching. Substantially linear ethylene polymers are commercially available from The Dow Chemical Company as Affinity[TM] polyolefin plastomers, which are produced using Dow's Insite[TM] technology, whereas homogeneous linear ethylene polymers are available from Exxon Chemical Company under the tradename Exact[TM].

The term 'interpolymer' is used herein to indicate a copolymer, terpolymer, or higher order polymer. That is,

EP 1 402 861 A1

at least one other comonomer is polymerized with ethylene to make the interpolymer. Interpolymers of ethylene are those polymers having at least one comonomer selected from the group consisting of vinyl esters of a saturated carboxylic acid wherein the acid moiety has up to 4 carbon atoms, unsaturated mono- or dicarboxylic acids of 3 to 5 carbon atoms, a salt of the unsaturated acid, esters of the unsaturated acid derived from an alcohol having 1 to 8 carbon atoms, and mixtures thereof.

If employed uncompounded, the ethylene to unsaturated carboxylic comonomer weight ratio is preferably greater than about 3:1, more preferably about 2:1. Hence, the comonomer concentration is preferably greater than 30 wt-%, more preferably greater than 33 wt-% and most preferably greater than 35 wt-%, with respect to the total weight of the ethylene interpolymer. The melt index of the interpolymers of ethylene may range from about 50 to about 2000, preferably from about 100 to 1500, more preferably from about 200 to 1200, and most preferably from about 400 to 1200 g/10 min. When employing a polymer having too low of a melt index uncompounded, the strength of the polymer tends to constrain the swelling of the particles of super absorbent material.

Suitable ethylene/unsaturated carboxylic acid, salt and ester interpolymers include ethylene/vinyl acetate (EVA) ethylene/acrylic acid (EEA) and its ionomers; ethylene/methacrylic acid and its ionomers; ethylene/methyl acrylate (EMA); ethylene/ethyl acrylate; ethylene/n-butyl acrylate (EnBA); as well as various derivatives thereof that incorporate two or more comonomers.

Other suitable thermoplastic polymers that may be employed include polylactide, caprolactone polymers, and poly (hydroxy-butyrate/hydroxyvalerate), certain polyvinyl alcohols, biodegradable copolyesters such as Eastman Copolyester 14766 (Eastman), linear saturated polyesters such as Dynapol or Dynacoll polymers from Huls, poly (ethylene oxide) polyether amide and polyester ether block copolymers available from Atochem (Pebax$^{TM}$) or Hoechst Celanese (Rite-flex$^{TM}$) respectively, and polyamide polymers such as those available from Union Camp (Unirez$^{TM}$) or Huls (Vestamelt$^{TM}$) or EMS-Chemie (Griltex$^{TM}$). Other suitable thermoplastic polymers are e.g. polyurethanes, poly-ether-amides block copolymers, polyethylene-acrylic acid and polyethylene-methacrylic acid copolymers, polyethylene oxide and its copolymers, ethylene acrylic esters and ethylene methacrylic esters copolymers, polylactide and copolymers, polyamides, polyesters and copolyesters, polyester block copolymers, sulfonated polyesters, poly-ether-ester block copolymers, poly-ether-ester-amide block copolymers, ionomers, polyethylene-vinyl acetate with a vinyl acetate content of at least 28% by weight, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, poly-caprolactone and copolymers, poly glycolide, polyglycolic acid and copolymers, polylactic acid and copolymers, polyureas, polyethylene, polypropylene, or mixtures thereof.

Particularly suitable preferred thermoplastic polymers are selected from thermoplastic poly-ether-amide block copolymers (e.g. Pebax$^{TM}$), thermoplastic poly-ether-ester-amide block copolymers, thermoplastic polyester block copolymers (e.g. Hytrel$^{TM}$), thermoplastic polyurethanes (e.g. Estane$^{TM}$), or mixtures thereof.

The polymeric base material for use herein preferably further comprise suitable compatible plasticizers. Plasticizers or mixture thereof are present in amounts ranging from 0% to 90%, preferably from 5% to 90%, more preferably 10% to 80%, even more preferably from 15% to 70% and most preferably from 30% to 65%, with respect to the total weight of the thermoplastic polymeric base material. Suitable 'plasticizers' for use herein generally will include any conventional plasticizers which decrease hardness and modulus, enhance pressure sensitive tack and reduce melt and solution viscosity. It is preferred that the plasticizer be water soluble or water dispersible or alternatively be a wax-like substance such as polyethylene or polypropylene glycol, glycerin, glycerol and its esters, butylene glycol or sorbitol. Other plasticizers suitable for use herein are esters of sucrose; phthalate plasticizers such as dioctyl phthalate and butyl benzyl phthalate (e. g., Santicizer 160 from Monsanto); benzoate plasticizers such as 1,4- cyclohexane dimethanol dibenzoate (e.g., Benzoflez 352 from Velsicol), diethylene glycol/dipropylene glycol dibenzoate (e.g., Benzoflez 50 from Velsicol), and diethylene glycol dibenzoate where the mole fraction of hydroxyl groups which have been esterified ranges from 0.5 to 0.95 (e.g., Benzoflex 2-45 High Hydroxyl also from Velsicol); phosphite plasticizers such as t-butyl diphenyl phosphate (e.g., Santicizer 154 from Monsanto); adipates; sebacates; epoxidized vegetal oils; polymerised vegetal oils; polyols; phthalates; liquid polyesters such as Dynacol 720 from Huts; glycolates; p-toluene sulfonamide and derivatives; glycols and polyglycols and their derivatives; sorbitan esters; phosphates; monocarboxylic fatty acids ($C_8$-$C_{22}$) and their derivatives; liquid rosin derivatives having Ring and Ball hydrocarbon oils which are low in aromatic content and which are paraffinic or naphthenic in character and mixtures thereof. Plasticizer oils are preferably low in volatility, transparent and have as little color and odor as possible. An example of a preferred plastizer is Carbowax$^{TM}$ polyethylene glycol from Union Carbide. The use of plasticizers also contemplates the use of olefin oligomers, low molecular weight polymers, vegetable oils and their derivatives and similar plasticizing liquids.

Particularly preferred plasticizers to be used herein are hydrophilic plasticizers such as acids, esters, amides, alcohols, polyalcohols, or mixtures thereof, among which even more preferred are citric acid esters, tartaric acid esters, glycerol and its esters, sorbitol, glycolates, and mixtures thereof, as disclosed in our application WO 99/64505. Said preferred hydrophilic plasticizers have a particularly high polar character and provide the further advantage that they do not impair, and possibly can even enhance, the moisture vapour permeability of the resulting layer formed from the polymeric base material and thus the liquid absorbent thermoplastic composition used herein comprising said preferred plasticizer or blend of plasticizers, when compared to a corresponding layer formed from an liquid absorbent thermoplastic composition comprising the same components, but without such a plasticizer or plasticizers.

These particularly preferred hydrophilic plasticizer or blend of hydrophilic plasticizers can of course also adjust the viscosity of the polymeric base material and thus of the liquid absorbent thermoplastic composition to desirable values in order to help processable when adhering said composition onto a substrate.

According to a further even more preferred embodiment of the present invention, for avoiding migration of the plasticizer or blend of plasticizers from the matrix of thermoplastic polymeric base material it is preferable that the molecular weight (MW) of the selected plasticiser or plasticisers be greater than 300, preferably greater than 1000, and more preferably greater than 3000. Plasticisers having a MW of at least 6000 work particularly well.

As a matter of fact absorbent articles are assembled by and contain conventional hydrophobic hot melt adhesive which can interact with the absorbent material of the present invention and particularly with its hydrophilic plasticizer giving rise to a degradation of the adhesive characteristics of the conventional hot melt adhesive. For example, a sanitary napkin or a panty liner is generally made of a liquid permeable topsheet, of a liquid impermeable backsheet, and of an fibrous absorbent core therebetween. Generally the outside of the backsheet is provided with stripes of conventional hot melt pressure sensitive adhesive for fastening the absorbent article to the user's panty. If said conventional absorbent core is substituted in total or in part by an absorbent element made of the absorbent thermoplastic composition described herein, for example coated onto the inner surface of the backsheet, the inventors have surprisingly found that if the hydrophilic plasticiser or the blend of hydrophilic plasticisers are selected from those having a molecular weight (MW) greater than 300, preferably greater than 1000, more preferably greater than 3000, there is no interference with the adhesive for fastening the absorbent article, i.e. no peel reduction of said adhesive is experienced, while using hydrophilic plasticizers of lower molecular weight a significant reduction of adhesive peel is experienced, presumably due to plasticiser migration out of the liquid absorbing thermoplastic composition.

Most preferably, plasticisers having the preferred molecular weight as explained above can be selected from the group consisting of glycerol esters, sucrose esters, sorbitol, epoxidized vegetal oils, polymerised vegetal oils, polyalcohols, polyols, liquid polyesters, p-toluene sulfonamide and derivatives, polyglycols and their derivatives, monocarboxylic fatty acids ($C_8$-$C_{22}$) and their derivatives, and mixtures thereof, wherein polyglycols and their derivatives are particularly preferred (i.e. polyethylene glycols and polypropylene glycols).

The polymeric base material for use in the liquid absorbent thermoplastic composition herein optionally also comprises tackifying resins. Tackifying resins are preferably present in amounts ranging from about 0% to 100%, more preferably 1% to 30%, even more preferably from 5% to 20%, most preferably from 8% to 15%, with respect to the total weight of the thermoplastic polymeric base material. As used herein, the term 'tackifying resin' means any of the liquid absorbent thermoplastic compositions described below that are useful to impart tack to the polymeric base material. ASTM D1878-61T defines tack as "the property of a material which enables it to form a bond of measurable strength immediately on contact with another surface". Tackifying resins comprise resins derived from renewable resources such as rosin derivatives including wood rosin, tall oil and gum rosin as well as rosin esters, natural and synthetic terpenes and derivatives of such. Aliphatic, aromatic or mixed aliphaticaromatic petroleum based tackifiers are also useful in the invention. Representative examples of useful hydrocarbon resins include alpha-methyl styrene resins, branched and unbranched $C_5$ resins, $C_9$ resins and $C_{10}$ resins, as well as styrenic and hydrogenated modifications of such. Tackifying resins range from being a liquid at 37°C to having a ring and ball softening point of about 135°C. Suitable tackifying resins for use herein include natural and modified resins; glycerol and pentaerythritol esters of natural and modified resins; polyterpene resins; copolymers and terpolymers of natural terpenes; phenolic modified terpene resins and the hydrogenated derivatives thereof; aliphatic petroleum resins and the hydrogenated derivatives thereof; aromatic petroleum resin and the hydrogenated derivatives thereof; and aliphatic or aromatic petroleum resins and the hydrogenated derivatives thereof, and combinations thereof. Commercial examples of these types of resins include Foral™ hydrogenated rosin ester, Staybelite™ hydrogenated modified rosin, Polypale™ polymerized rosin, Permalyn™ rosin ester, Pentalyn™ rosin ester, Adtac™ oil extended hydrocarbon resin, Piccopale™ aromatic hydrocarbon, Piccotac™, Hercotac™ aromatic modified aliphatic hydrocarbon, Regalrez™ cycloaliphatic resins, or Piccolyte™ from Hercules, Eselementz™ from Exxon Chemical aliphatic hydrocarbon and cycloaliphatic resins, Wingtack™ from Goodyear Tire & Rubber Co. synthetic polyterpene resins including aromatic modified versions, Arkon™ partially and fully hydrogenated aromatic resins from Arakawa Chemicals, Zonatac™ styrenated terpene resin, Zonarez™ rosin ester and Zonester™ rosin ester from Arizona Chemical and Nevtac™ aromatic modified aliphatic hydrocarbon from Neville Chemical Company.

The polymeric base material for use in the liquid absorbent thermoplastic composition herein optionally also comprises anti-oxidants. Antioxidant are typically present in amounts ranging from about 0% to 10%, preferably 0.2% to 5%, more preferably from 0.5% to 2%, most preferably from 0.75% to 1.5%, with respect to the total weight of the thermoplastic polymeric base material. Suitable 'anti-oxidants' for use herein include any conventional anti-oxidants, and are preferably hindered phenols such as for example Ethanox 330™ 1,3,5-trimethyl-2,4,6-tris(3,5-di-tert-butyl-4-hydroxybenzyl) benzene which is commercially available from the Ethyl Corporation. Other examples for suitable anti-oxidants are hindered phenolics (e. g., Irganox 1010, Irganox 1076).

The polymeric base material for use in the liquid absorbent thermoplastic composition herein optionally also comprises surfactants. Suitable 'surfactants' for use herein are additives that reduce the surface tension and/or contact angle of the polymeric base material. Surfactants are typically present in amounts ranging from about 0 wt-% to about 25 wt-% and preferably from about 5 wt-% to about 15 wt %, with respect to the total weight of the thermoplastic polymeric base material. Suitable surfactants include nonionic, anionic, and silicone surfactants. Exemplary nonionic surfactants are: Ethoxylates of (i) $C_1$-$C_{18}$, preferred $C_8$-$C_9$ alkyl or dialkyl phenols, such as those sold under the tradenames Macol DNP-10, available from PPG Industries, Gurnee, III., a 10 mole ethoxylate of dinonyl phenol, and Triton X-100, available from Union Carbide, a 10 mole ethoxylate of octyl phenol ; (ii) alkyl $C_8$-$C_{60}$ monoalcohols, such as those sold under the tradenames Surfonic L-12-8, an 8 mole ethoxylate of dodecanol, available from Huntsman Chemical Co., and Unithox 480, a 38 mole ethoxylate crystalline surfactant available from Petrolite Specialty Polymers Group, Tulsa, Okla.; and (iii) propylene oxide polymers, such as those sold under the tradename Pluronic, which are ethylene oxide/propylene oxide block copolymers having a Mn of 200 to 3000, available from BASF; and benzoates formed by partial condensation of benzoic acid with hydrophilic di or mono-ols having less than 1000 Mn, such as the product of condensing about three equivalents of benzoic acid with four equivalent of diethylene glycol, commercially available as XP 1010 from Velsicol Chemical. A preferred nonionic surfactant blend is Atmer 685, available from ICI Surfactants (Wilmington, DE). Suitable anionic surfactants are: $C_8$-$C_{60}$ alkyl ethoxylate sulfonates, $(CH_3$-$(CH_2)_{11-14}$-$(O$-$CH_2$-$CH_2)_3$-$SO_3^-$ $Na^+$, such as, Avenel S30, available from PPG Industries; alkyl $C_8$-$C_{60}$ sulfonates, such as, Rhodapon UB ($C_{12}$-$SO_3^-$ $Na^+$) available from Rhone Poulenc; and alkyl/aromatic sulfonates, such as those sold under the tradename Calsoft. Suitable silicone surfactants are ethoxylates or propoxylates of polydimethyl siloxane, having a number average molecular weight of 500 to 10,000, preferably 600 to 6000, such as are sold under the tradenames Silwet L-77, L-7605, and L-7500 availablefrom OSi Specialties, Danbury, Conn.; and product 193 from Dow Corning. The preferred surfactants are those with lower molecular weights because these have increased compatibility in the polymeric base material. The maximum acceptable molecular weight depends on the type of surfactant and the other ingredients in the polymeric base material and thus the liquid absorbent thermoplastic composition.

Other optional components of the polymeric base material for use herein include anti-ultraviolets, dyes, antibacterials, odour adsorbing materials, perfumes, pharmaceuticals, and mixtures thereof, which may be present within the liquid absorbent thermoplastic compositions at a level of up to 20% by weight of the composition.

The liquid absorbent thermoplastic composition suitable for use herein is preferably a hot-melt adhesive, i.e. the polymeric base material comprises a hot-melt adhesive, which is capable of absorbing aqueous liquids. Such preferred liquid absorbent thermoplastic compositions comprise (by weight):

a) from about 5% to about 99% of a polymeric base material, comprising
a') from about 10% to about 50% of a block copolymer,
a") from about 0% to about 50% of a tackifying resin;
a"') from about 10% to about 80% plasticizer,
a"") from about 0% to about 2.0% antioxidant; and
b) from about 1% to about 95% of particles of water insoluble water swellable absorbent material.

While the absorption characteristics (e.g. the liquid absorption in terms of grams of liquid absorbed for each gram of composition) of the liquid absorbent thermoplastic composition suitable to use herein can be adjusted by varying the percentage and the type of absorbent material contained in the polymeric base material/matrix, preferably the liquid absorbent thermoplastic composition for use herein has a liquid absorption of at least 2 g/g of a saline solution, such as a NaCl 0.9% water solution, preferably of at least 7 g/g, more preferably of at least 10 g/g.

The liquid absorption of the liquid absorbent thermoplastic composition can be evaluated according to the Water Absorption Test described herein, made on the material in form of a film sample 76.2 mm long by 25.4 mm wide by 0.2 mm thick, and using the saline solution mentioned above instead of distilled water.

Highly preferred thermoplastic polymeric base materials for use in the liquid absorbent thermoplastic compositions described herein before are those having a water absorption capacity of at least greater than 30%, preferably greater than 40%, more preferably greater than 60% and most preferably greater than 90%, when measured according to the Water Absorption Test described herein in accordance with ASTM D 570-81, on a film 200 μm thick. The intrinsic absorbency of the polymeric base material/matrix allows for a more effective diffusion of the body fluid within the matrix and, consequently, for a better spreading of the body fluid which can reach a greater number of absorbent material particles which in turn give rise to a better utilization of the absorbent material.

Highly preferred absorbent thermoplastic compositions described herein before are those showing good integrity in wet state and hence having a tensile strength in wet state which is at least 20%, preferably at least 40%, and more preferably 60% of the tensile strength of said composition in dry state. Said tensile strengths are evaluated according to the Tensile Strength Test described herein. It should be appreciated that by selecting a thermoplastic base material, in the liquid absorbent thermoplastic composition herein having a higher value of water absorption, the absorbent composition will have better liquid absorption/handling characteristics, while not compromising on tensile strength in wet state. Indeed such absorbent composition will remain substantial intact and have sufficient tensile strength for its intended use, also upon liquid absorption.

Indeed the highly preferred liquid absorbent thermoplastic compositions for use herein offer improved mechanical and absorbent properties. Without to be bound by theory it is believed that the intrinsic absorbency of the matrix allows the body fluid to be acquired and diffused within the matrix thus permitting fluid contact with the absorbent material contained in the matrix and their swelling, without the necessity of having a matrix of low cohesive strength but with a matrix which remains substantially intact and having sufficient strength upon fluid absorption.

The absorbent material or mixture thereof in particle form are blended with the polymeric base material in any known manner to provide the liquid absorbent thermoplastic composition for use herein. For example, by first melting the thermoplastic polymeric base material and then by adding and mixing the required amount of absorbent particles. Indeed the liquid absorbent thermoplastic composition for use herein especially those in the form of a hot-melt adhesive may be formed by blending the polymeric base material (block copolymer and preferably plastifier) and the absorbent material in suitable adhesive processing equipment, such as a melt mixer or extruder. Preferably the liquid absorbing thermoplastic compositions for use herein are formulated to have hot melt characteristics so that they can be applied utilizing any know method used for applying hot melt adhesives. Method of coating thermoplastic composition continuously onto a substrate acting as a support layer are as described in PCT application WO 96/25902 or WO96/38114. Such methods can be applied herein.

At least at the coating temperature, since the liquid absorbent thermoplastic composition comprises thermoplastic polymeric base materials, it can exhibit adhesive properties on a supportive substrate in order to form a composite structure such that no additional adhesive is required to achieve a permanent attachment between the absorbent element provided partially or preferably completely per the liquid absorbent thermoplastic composition, and the substrate. However, while hot melt techniques are preferred, any other known method for processing thermoplastic compositions can be used for processing the absorbent compositions in any known form/pattern. Also, any known method for spraying, printing, dotting, coating or foaming thermoplastic compositions can be used as well as extrusion, lamination processes.

Particularly suitable methods for applying the liquid absorbent thermoplastic composition to a substrate is per gravure printing or slot coating. Both methods are particularly suitable for discontinuous application of the thermoplastic composition described herein onto a substrate.

The total area of a surface onto which the liquid absorbent thermoplastic composition is applied to (preferably backsheet or any intermediate layers like fluid distribution layer if present) defines the actual absorbent surface of said composition in the absorbent article.

Because the absorbent composition in the preferred embodiment herein is thermoplastic, it allows for hot melt technique applications which in turn increase the versatility of its application. The liquid absorbent composition can be provided covering continuously or discontinuously the whole surface area or only a region of the substrate to which it is applied to, for example the central region of an article where body fluid is discharged in use. Indeed in a preferred embodiment of the present invention the liquid absorbent thermoplastic composition is configured in a plurality of unattached spaced apart storage zones, i.e. in two or more unattached zones which are individualized adjacent zones separated from each other without any physical link. The overall system of unattached spaced apart zones can be positioned in desired patterns. There is a discrete separation distance between adjacent unattached spaced apart zones. Typically each zone extends substantially continuously over an area of not less than 0.001 cm$^2$, preferably from 0.01 to 10 cm$^2$ and more preferably from 1 to 4 cm$^2$. Typically the spacing distance between two adjacent zones is at least 0.1 mm, preferably is at least 1 mm, more preferably from 1 to 5 mm. Each zone may take any shape or size, they might be regular or irregular in shape, in the form of dotes, stripes, rectangles, squares and so on. Said zones might have all the same shape and dimensions or said zones might have different shapes and/or different dimensions. In addition the overall system of storage zones can be positioned and arranged in desired patterns to provide a selected operable zones array composed of the cooperating individual unattached spaced apart storage zones. The zones array, in one aspect of the present invention, can provide a pattern size having total surface area extend of not less than 1 cm$^2$, preferably from 1 to 100 cm$^2$ and most preferably from 10 to 50 cm$^2$. By 'pattern size' it is meant herein total surface area covered per all unattached spaced apart zones without considering the interstitial area between said zones.

In a preferred embodiment the article comprises as said unattached spaced apart zones, elongated zones also called stripes. In a preferred embodiment herein the elongated zones individually preferably have a length which is at least 10%, preferably at least 30%, more preferably at least 50% of the maximum length of the article taken in longitudinal direction (LD) and width which is from 1% to 5% of the maximum width of the article taken in transverse direction (TD i.e. D-D' in Figure 1). A highly preferred execution is illustrated in Figure 1. Such executions are highly preferred herein as they provide outstanding flexibility characteristics in both transverse direction and longitudinal direction. Indeed Figure 1 shows a pantyliner with a topsheet and backsheet being coextensive with-each other and being attached to each other along the outer edge of the pantyliner per heat bonding C. This pantyliner comprises a storage element with storage zones A that take the form of stripes delimited per interstitial void regions B. The width 'a' of the stripe A can vary as desired. In Figure 1 the width 'a' is about 2 mm and the distance 'b' separating two adjacent stripes A is about 2 mm. Figure 2 is a cross section of the pantiliner of Figure 1 taken trough line D-D', said liner comprises a topsheet (1), an adhesive layer (2) for adhering the topsheet to the underlying fluid distribution layer (3), a storage element (4), a backsheet (5) and adhesive layer (6) for adhering the backsheet (5) of the liner to a release liner (7).

If desired although not preferred an optional component for inclusion in the absorbent element according to the present invention is a fibrous layer adjacent to, and typically underlying the storage layer. If present this layer provides a substrate on which to deposit the liquid absorbent thermoplastic composition during manufacture of the absorbent element. If present such a layer typically provides some additional fluid-handling capabilities such as rapid wicking of fluid along the length of the absorbent article but might compromise on the flexibility properties of the article.

Topsheet

The topsheet is compliant, soft feeling, and non-irritating to the wearer's skin. The topsheet also can be elastically stretchable in one or two directions. Further, the topsheet is fluid pervious permitting fluids (e.g., menses and/ or urine) to readily penetrate through its thickness. A suitable topsheet can be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers.

Preferred topsheets for use in the present are selected from high loft nonwoven topsheets and apertured formed film topsheets. Apertured formed films are especially preferred for the topsheet because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. 5,006,394. Particularly preferred microapetured formed film topsheets are disclosed in U.S. patent 4,609,518 and U.S. patent 4,629,643. The preferred topsheet for the present invention is the formed film described in one or more of the above patents and marketed on sanitary napkins by The Procter & Gamble Company of Cincinnati, Ohio as "DRI-WEAVE."The body surface of the formed film topsheet can be hydrophilic so as to help liquid to transfer through the topsheet faster than if the body surface was not hydrophilic. In a preferred embodiment, surfactant is incorporated into the polymeric materials of the formed film topsheet such as is described in U.S. Patent Application Serial No. 07/794,745, filed on November 19, 1991. Alternatively, the body surface of the topsheet can be made hydrophilic by treating it with a surfactant such as is described in the above referenced U.S. 4,950,254.

Backsheet

The backsheet prevents the exudates absorbed and contained in the absorbent element from wetting articles that contact the article such as pants, pajamas and undergarments. The backsheet is impervious to liquids (e.g., menses and/or urine) and is preferably manufactured from a thin plastic film, although other flexible liquid impervious materials can also be used. The backsheet needs to be compliant and will readily conform to the general shape and contours of the human body. The backsheet also can have characteristics allowing it to elastically stretch in one or two directions.

The backsheet can comprise a woven or nonwoven material, polymeric films such as thermoplastic films of polyethylene or polypropylene, or composite materials such as a film-coated nonwoven material.

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further, the backsheet can permit vapors to escape from the absorbent structure (i.e., be breathable) while still preventing exudates from passing through the backsheet.

The backsheet typically forms the garment facing surface on which the panty fastening adhesive is placed. Panty-fastening-adhesives can comprise any adhesive or glue used in the art for such purposes with pressure-sensitive adhesives being preferred. Suitable non-extensible adhesives are Century A-305-IV manufactured by the Century Adhesives Corporation, Instant Lock 34-2823 manufactured by the National Starch Company, 3 Sigma 3153 manufactured by 3 Sigma, and Fuller H-2238ZP manufactured by the H.B. Fuller Co. Suitable adhesive fasteners are also described in U.S. Patent 4,917,697. Suitable elastically stretchable adhesive films are such as Findley adhesive 198-338, or an elastically stretchable adhesive film known as 3M XPO-0-014 available from the Minnesota Mining and Manufacturing Company of St. Paul, Minnesota; or spray adhesives such as 3M adhesive 1442 on a low modules elastic film. Other suitable panty-fastening-adhesives are shown in PCT International Patent Publication No. WO 92/04000; WO 93/01783 and WO 93/01785.

The thickness of a preferred embodiment of the present invention especially for panty liners is less than 3 mm, more preferably in the range of 0.5 to 1.5 mm and even most preferably in the range of 0.7 to 1.2 mm according to the thickness measurement method described herein below.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## STIFFNESS TEST METHOD

The procedure for measuring the flexibility/drapability of the absorbent article is as follows:

PRINCIPLE

This test allows the measurement of the flexibility of a given absorbent article (or of a portion thereof) in different environments (i.e. room condition and human body condition).

GENERAL COMMENTS

The flexibility test is only one way of measuring a sample's flexibility and is believed to be one of the components which users of absorbent articles often refer to as conformability. This measurement method should be followed as closely as possible and should not be confused with the multidirectional flexibility described in US 5,009,653. The test is carried out either in longitudinal direction (machine direction MD) or in transverse direction (cross direction CD). It is to be understood herein that a given test sample is measured in same direction when comparing its flexibility at 38°C and 23°C. According to the present invention a difference in flexibility of at least 20% more, when comparing flexibility for a given sample at 23°C to its flexibility at 38°C is meet if this is achieved in at least one direction, for example longitudinal direction or transverse direction. In preferred embodiment herein this difference is present in both directions.

APPARATUS

AB Lorentzen & Wettre Stockolm, Sweden - Stiffness Tester app 16 Type 10-1 n 734 This stiffness tester measures the flexibility of a sample by folding it for a certain angle (up to 30°). The cell (knife) measures the resistance of the sample to the folding.

Conditioned Room:A room conditioned to 23°C + 2 °C, 50 % + 2 % Relative Humidity

Climatic chamber, suitable to contain the stiffness tester and the test samples is conditioned to 38°C + 2°C, 75 %+ 2 % Relative Humidity to simulate body temperature

Talcum Powder, scissors

STIFFNESS TESTER SETTING

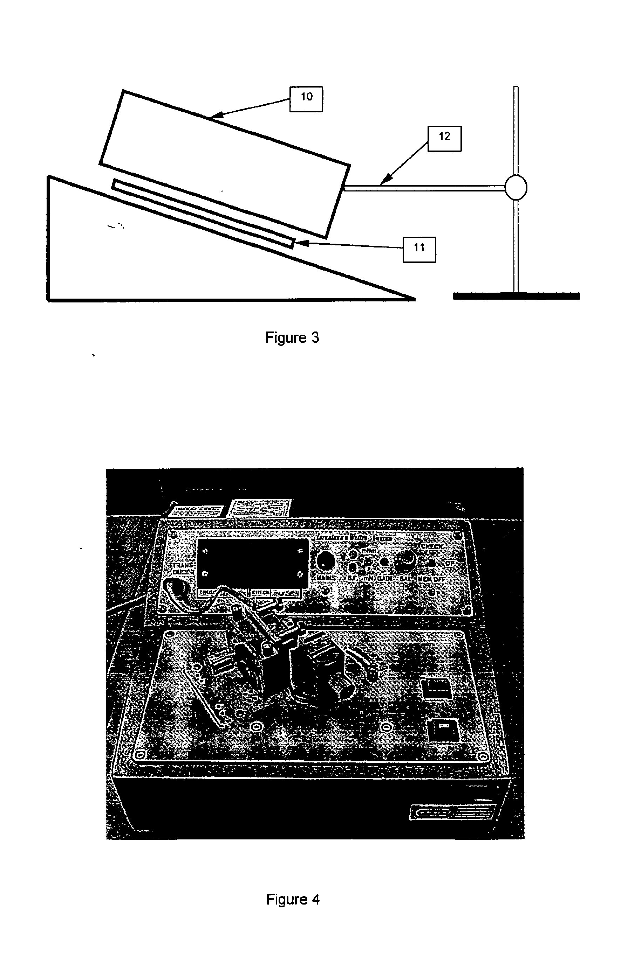

Set distance at 5mm using the screws for bending length
Set rotation at 30° using the screw for bending angle
Set measure units to mN
Check calibration with the switch "CHECK" (see Figure 4) and compare with standard value labeled on the instrument. If needed screw the gain "BAL" (see Figure 4) until reaches 0.
Put the knob switch on forward.

SAMPLE PREPARATION

The absorbent articles and resulting test samples should be stressed as little as possible during manipulation to have minimal impact on flexibility measurement per such manipulation. In general, a single test sample should be tested only one time. Absorbent articles for use in this test must be very carefully handled to prevent folds, wrinkles, bends, etc. This test is intended to be used on non folded absorbent articles. If only folded articles are available, the flexibility can be measured by taking test samples between the fold lines. The test should be used on complete test samples, i.e. with all layers having the same shape extending to the complete sample surface and fully glued together.

1. Remove the release liner from absorbent article and deactivate the panty fastening adhesive (PFA) by carefully powdering the PFA with the minimum amount of talcum sufficient to avoid sticking. Blow out the remaining talcum from the article.

2. Cut 2 samples using a scissors out of core zone of the article (e.g., pantiliner). The test sample has to be cut lengthwise from the center of the absorbent article to be square without crimp and the longitudinal direction/machine direction should be identified (see below diagram A). Sample dimensions for measurements are 38 mm x 38 mm = 14.44 cm$^2$.

## Diagram A

Long. Direct.  Long. Direct.

In case of folded articles (typical single packaged article in wrapper) cut 1 sample per article in the central zone excluding any folding and identify the Machine Direction. For 3 folded articles, twice as much articles are needed to have same number of samples.

TEST PROCEDURE

The flexibility of the article is measured at two different temperatures with same test method except for temperature condition, i.e. first at room temperature and then at body temperature. To minimize differences due to variability, it is recommended to use one of the 2 samples of the same article for each test condition, i.e. one for room conditions and one for body temperature conditions.
The stiffness-tester should be placed on a bench directly in front of the operator. It is important that the bench is relatively free of vibration, that there is no air flow during the measurement and that the bench is free of draft.

Room temperature (23°C - 50 % RH):

The samples to be tested are acclimatized before testing for at least 4 hours. Each sample is put in the clamp as showed in Figure 4 with longitudinal direction (LD) in horizontal. The sample is screwed very delicately avoiding any compression to ensure that the sample is not crushed. The knife is moved towards the sample (load cell) until it makes contact and the operator verifies that the display displays 0 mN.
The start button is switched on to measure the flexibility of the sample. The display will show the flexibility in mN during the test and right after it. The value is recorded when the clamp returns back and the value on the display remains constant.

Body temperature (38°C - 75 % RH):

All samples and equipment must be put in the climatic chamber for 1 h before testing at 38°C - 75 % RH. After 1 h the instrument and samples to be tested are taken out from the climatic chambers and the following procedure is immediately carried out. Flexibility value for a given test sample is obtained in less than 1 minute after having taken the test sample out of the climatic chamber.

The sample is put in the clamp as showed in Fig.4 with longitudinal direction (MD) in horizontal. It is screwed very delicately avoiding any compression to secure that the sample will not crush.The knife (load cell) is moved towards the sample until it makes contact and the operator verifies that the display displays 0 mN. The start button is switched on to measure the rigidity of the sample. The display will show the rigidity in mN during the test and right after it. the value is recorded when the clamp returns back and remains constant on the display.

DATA REPORT:

The equation used to express Flexibility according to the present invention is as follows:

$$Flexibility = mN$$

To have a statistical significance it is recommended to test 12 samples (six topsheet side and six backsheet side) of a given absorbent article (to make statistic evaluation we need minimum 6 samples per article option). The average and the standard deviation for each article tested and test significance is reported. Test statistically the population means. Two different articles's flexibility are considered significantly different if the confidence is >95%.

## Thickness measurement

The thickness should always be measured at the thickest possible place, usually in the center of the absorbent article. For convenience the measurement is conducted on the absorbent article inclusive any protective cover means present. The article should be reconditioned at 50 % humidity and 23° C for two hours within its usual package and be removed not more than five minutes prior to the measurement.

The thickness is measured with a micrometer gauge having a range of 0 to 30 mm and capable of plus minus 0.5 mm tolerance. The gauge must not be spring loaded and should have a foot moving downwards under gravity. The micrometer foot has a diameter of 40 mm and is loaded with a 80 gram weight. The measurement is taken between 5 and 10 seconds after the foot has been lowed to come into contact with the absorbent article. Measurements should be taken often enough to allow statistical analysis to determine average thickness within a sigma of plus minus 0.1 mm. A detailed description of the thickness measurement can also be found in US-Patent 5, 009, 653.

## Water Absorption Test

The determination of liquid absorption capacity of thermoplastic polymeric base material and liquid thermoplastic absorbent composition used in preferred embodiment herein is conducted according to the standard test method ASTM D 570-81 with the following conditions. The measurement of absorption for thermoplastic polymeric base material and liquid thermoplastic absorbent composition is made on the material in form of a film sample 76.2 mm long by 25.4 mm wide by 0.2 mm thick. For all materials a 24 hours immersion in liquid at 23°C is chosen and the percentage of liquid absorbed in accordance with the ASTM D 570-81 standard is reported. The liquid is distilled water for thermoplastic polymeric base material and 0.9% NaCl water solution for liquid absorbent thermoplastic compositions comprising the matrix of thermoplastic polymeric base material with particles of absorbent material dispersed therein.

## Tensile Strength Test

The test measures the mechanical resistance of a sample of material as tensile strength at break, according to the standard test method ASTM D 412-92, under the following conditions. The test is performed on samples made of the liquid absorbent thermoplastic composition and having a length of 130 mm, a width of 25.4 mm, and a thickness of 2 mm, and being continuous, obtained with any suitable method, for example by pouring the liquid absorbent thermoplastic composition in molten state at a suitable temperature, e.g. 180°C into a metallic pan lined with release paper in a continuous layer having a thickness of 2 mm, and then after cooling cutting from this layer the samples of the desired dimensions.

The test is performed on samples made of the same composition both in dry and in wet state. In order to prepare the samples in wet state a sample is placed in a container of a saline solution (e.g. 0.9% NaCl distilled water solution) maintained at a temperature of 23 ± 1°C, and shall rest entirely immersed for ten minutes. At the end of ten minutes, the sample shall be removed from the water, all surface water wiped off with a dry cloth, and tested for wet tensile strength as provided in the standard test method.

Sample preparation

When starting from an absorbent article comprising the liquid absorbent thermoplastic composition in turn comprising the matrix of thermoplastic polymeric base material with particles of water-insoluble water-swellable absorbent material dispersed therein, for example a disposable absorbent article with the thermoplastic composition coated onto a substrate, the thermoplastic composition can be isolated with known means in order to be tested. Typically in a disposable absorbent article the topsheet is removed from the backsheet and both are separated from any additional layers if present. The liquid absorbent thermoplastic composition is scraped with a spatula from its substrate layer. The recovered thermoplastic composition will be used to prepare samples as mentioned above with known means. For example, the thermoplastic composition can be melted, or dissolved with a suitable solvent. Particles of absorbent material can be also separated from the thermoplastic composition, in order to isolate the thermoplastic polymeric base material, as it is known in the art, for example by suitably sieving or filtering from the molten state, or preferably from the solution.

**Dunk absorption capacity test for absorbent article**

*Purpose*

This method measures the weight of a test fluid (Synthetic Urine B) which is retained in an absorbent article after it is immersed in the test fluid for a certain period of time and after applying a static pressure of 17 g/cm$^2$ on the article for 2 minutes.

*Equipment*

| Item | Supplier |
| --- | --- |
| Magnetic stirrer, with a top plate of 100 mm diameter | IKAMAG or equivalent |
| Magnetic stirring bar 64 mm | IKAMAG or equivalent |
| Beaker, 2000 ml capacity | Hirshmann or equivalent |
| Digital balance - accurate ± 0.01 g | Mettler or equivalent |
| Timer control (stop watch) accurate to 0.5 sec | Mali or equivalent |
| Plexyglas plate and basin (at least 2 cm deep), at least 25x25 cm | Fisher or equivalent |
| Plexyglas slope with 15° angle to the horizontal, at least 8 cm wide | Convenient source |
| Weight of 1320 (± 15 g) with base dimensions of 15.5 x 5 cm (equivalent to 17 g/cm$^2$) with a foam base covered with plastic film | Convenient source |
| Weight of 2265 g (± 15g) with base dimensions of 20.5 cm x 6.5 cm with a foam base covered with plastic film (for absorbent article with large dimensions) | Convenient source |
| A stand with an extendable arm to fix the weight on the slope and stop it from sliding | Convenient source |

*Preparation of Synthetic Urine B solution*

Place a 2000 ml beaker on the balance. Weigh 800 g of demineralised water into the beaker. Carefully add into the beaker exact amounts of the following materials:

1) UREA ($NH_2CO\ NH_2$) 20.00 grams
2) Sodium Chloride (NaCl) 9.00 grams
3) Magnesium Sulfate ($Mg\ SO_47H_2O$) 1.10 grams
4) Calcium Chloride ($CaCl_22H_2O$) 0.79 grams

Fill up to 1000 ml with mineralized water and mix the chemicals with aid of stiming bar on a magnetic stirrer until solids are well dissolved (about 5 minutes). The resulting solution should be covered with parafilm to prevent evaporation or contamination.

*Test Procedure*

- Pre-weigh the absorbent article (including release paper) using the digital balance and record the 'initial dry weight' to an accuracy of ± 0.01 g.
- Fill the basin partially with Syn. Urine B solution and ensure that the depth of the solution in the basin throughout the test is maintained higher than 5 mm.
- Put the absorbent article face down (topsheet down) in the basin for 25 minutes.
- Gently remove the absorbent article from the basin by holding it from its edge. Allow it to drip for 2 seconds in a vertical position, then place it face down on the slope with angle 15°.
- Place the weight (10) gently on the article (11) for two minutes (± 5 sec) and use the arm (12) of the stand to fix the weight (Fig. 3).
- Remove the weight after the 2 minutes and hold the article from the edge and allow it to drip in a vertical position for two seconds, then re-weigh it and record the 'wet weight' to an accuracy of ±0.01 g.

The following precautions should be followed during the test to ensure consistent accuracy of the results:

- The absorbent article should be kept flat without bending or twisting in all steps of the test
- The article should be held gently at the top while the load is applied to prevent it from sliding. Applying the load should be done carefully and gradually starting from the top of the sample
- Ensure that the slope and base of the weight are dry before placement of the article. Also ensure that the balance tray is dry before weighing the wet pantiliner.

*Calculation of capacity (Dunk)*

$$Dunk (g) = wet weight (g) - the initial dry weight (g)$$

The liquid absorption capacity is reported in grams for a given absorbent article tested.
To have a statistical significance it is recommended to test 5 absorbent articles and average results obtained.

The present invention is illustrated per following examples:

Absorbent articles according to the present invention are described herein after:

Examples A and B

Panty liner A as illustrated in Figures 1 and 2 comprises an apertured polyethylene formed film topsheet (1) (code name X-28278 available from Tredegar), a spiral layer of adhesive (2) (D3151 available from Fuller), a fluid distribution layer made of nonwoven material (3) (carded polyethylen/polypropylene bico Sawabond VP40/01/11 available from Sandler), a storage element (4) consisting of the composition exemplified in below Example 1 (total amount 0.5 g / basis weight 152 g/m2 ), a plastic polyethylene film backsheet (5) without pigment (code 14/18020, available from RKW), stripes of panty fastening adhesive (6) (HL1461 available from Fuller) and a release liner (7).
The composition of example 1 is applied per printing in stripes on to the backsheet as per Figure 1 to cover 34.5% of the whole surface of total pantiliner. Each stripes as a width of 2 mm and is separated from adjacent stripes by 2 mm. Pantiliner A has a flexibility in CD direction of 54mN and in machine direction MD of 14 mN as per Stiffness test described herein at 23°C.
Panty liner B is the same as pantiliner A except that the composition of Example 1 covers the backsheet in continuous manner to cover 70% of the whole surface of the article.
Other pantiliners according to the present invention can be prepared as pantilyner A or B but with a storage element consisting of any of the compositions exemplified in Examples 2, 3 or 4.

Example 1

A liquid absorbent thermoplastic composition for use in the articles of present invention is following mixture, forming a hot-melt adhesive:

| | |
|---|---|
| 18% | Estane T5410 from Noveon |
| 17% | PEG E400 from Dow Chemical |
| 1% | Irganox B 225 from Ciba Speciality Chemicals |
| 19% | CR00 (former PM17) from Savare |
| 45% | Aquakeep 10 SH-NF® from Sumitomo-Seika. |

Estane T5410 is a polyurethane hydrophilic polymer, PEG E 400 is a polyethylene glycol (plasticizer, MW about 400), Irganox B 225 is an anti oxidant, CR 00 is a hot melt adhesive commercially available from Savare.

Example 2

A thermoplastic polyether-amide block copolymer available from Atofina (France) under the trade name Pebax MV 3000 is compounded with polyethylene glycol PEG 400 (plasticiser, MW about 400), Sodium Dodecyl Sulphate (SDS), both available from Aldrich Co., and Irganox B 225 (anti oxidant agent) available from Ciba-Geigy.
The formulation in percent by weight has the following composition, and constitutes the thermoplastic polymeric base:

| | |
|---|---|
| 28.6% | Pebax MV 3000 |
| 68.6% | PEG 400 |
| 1.4% | SDS |
| 1.4% | Irganox B 225 |

The thermoplastic polymeric base has a water absorption of 43%, value measured according to the Water Absorption Test described herein. The thermoplastic base is formed into a film to be used in the Water absorption Test by melt coating the thermoplastic base at a temperature of 180°C onto a release paper to obtain a film having the prescribed thickness of 200 µm. After cooling at room temperature the film is separated from the release paper.
A superabsorbent material in particle form (average particle size between 20 and 30 µm and being in form of spherical beads) sold under the trade name Aqua Keep 10SH-NF from Sumitomo Seika Chemical (Japan) is added to the thermoplastic polymeric base while maintained at a temperature of 180°C and uniformly dispersed, in an amount corresponding to 42.9% by weight of the thermoplastic base. The liquid absorbent thermoplastic composition has the following final composition by weight:

| | |
|---|---|
| 20% | Pebax MV 3000 |
| 48% | PEG 400 |
| 30% | Aqua Keep 10SH-NF |
| 1 % | SDS |
| 1% | Irganox B 225 |

Example 3

A thermoplastic polyether-ester block copolymer available from Du Pont (USA) under the trade name Hytrel 8171 is compounded with polyethylene glycol PEG 400 (plasticiser, MW about 400), polyethylene glycol PEG 1500 (plasticiser, MW about 1500), both available from Aldrich Co., and Irganox B 225 (anti oxidant agent) available from Ciba-Geigy.
The formulation in percent by weight has the following composition, and constitutes the thermoplastic polymeric base:

| | |
|---|---|
| 28.6% | Hytrel 8171 |
| 21.4% | PEG 400 |
| 28.6% | PEG 1500 |

(continued)

| 1.4% | Irganox B 225 |
|------|---------------|

The thermoplastic polymeric base has a water absorption of 96%, value measured according to the Water Absorption Test described herein. The thermoplastic base is formed into a film to be used in the Water absorption Test by melt coating the thermoplastic base at a temperature of 180°C onto a release paper to obtain a film having the prescribed thickness of 200 μm. After cooling at room temperature the film is separated from the release paper.

A superabsorbent material in particle form sold under the trade name Aqua Keep 10SH-NF is added to the thermoplastic polymeric base while maintained at a temperature of 180°C and uniformly dispersed, in an amount corresponding to 42.9% by weight of the thermoplastic base. The liquid absorbent thermoplastic composition has the following final composition by weight:

| | |
|------|-------------------|
| 20% | Hytrel 8171 |
| 15% | PEG 400 |
| 34% | PEG 1500 |
| 30% | Aqua Keep 10SH-NF |
| 1% | Irganox B 225 |

Example 4

Another liquid absorbent thermoplastic composition for use herein has the following final composition by weight:

| | | |
|--------------------------|---------------|-------------------|
| 18 % of Pebax MV 3000 | (polymer) | Elf Atochem |
| 32 % of Diacetine | (plasticizer) | Novachem Aromatici |
| 5% of Ketjenflex 8 | (plasticizer) | Akzo Nobel |
| 45% of Aquakeep 10 SH-NF | (agm) | Sumitomo Seika |

Diacetine is 1,2,3 Propanetriol Diacetate (plasticiser, MW about 176), Ketjenflex 8 is N-Ethyl-orto, para-Toluene-Sulfonamide (plasticiser, MW about 199).

All these compositions have respectively a tensile strength in wet state which is at least 35% of the tensile strength of the composition in dry state, when evaluated according to the tensile strength test described herein.

**DATA REPORT**

Below table reports the flexibility behavior of articles according to the present invention at 23°C and 38°C, namely Pantiliner A as described herein before, in comparison to articles representative of the prior art, namely Carefree Extra thin breathable (KC) and Sarasarty ex Kobayashi.

For each type of article to be tested 10 articles were respectively analyzed for their respective flexibility behavior. For each article 2 samples were prepared as described in test method described herein before: one was tested at room temperature condition (23°C) the other one was tested at body temperature condition (38°C) as described herein before. The average value obtained respectively out of the 10 samples tested under each condition are reported in table I below. The flexibility data reported below in table I is the one measured in transverse direction (i.e., cross direction CD).

The lower the flexibility value expressed in mN , the more flexible the article is.

Carefree Extra Thin Breathable pantiliner is a commercially available liner on European market (manufactor: Kimberly-Clark) with an absorbent element being made of cellulose, polyethylene and polypropylene fibers.

Sarasarty ex Kobayashi with an absorbent element made of synthetic fibers is commercially available on Japanese market.

TABLE I

| Article tested | Flexibility expressed in mN at 23°C CD | Flexibility expressed in mN at 38°C CD | Flexibility increase in % | Dunk capacity expressed in grams |
|----------------|----------------------------------------|----------------------------------------|---------------------------|----------------------------------|
| Pantiliner A | 54 | 35 | 35.2 | 7.0 |

TABLE I   (continued)

| Article tested | Flexibility expressed in mN at 23°C CD | Flexibility expressed in mN at 38°C CD | Flexibility increase in % | Dunk capacity expressed in grams |
|---|---|---|---|---|
| Carefree | 97 | 88 | 9.3 | - |
| Sarasarty | 37 | 31 | 16.2 | - |

**Claims**

1. A flexible absorbent article suitable for use with garment, **characterized in that** said article has a flexibility when measured according to stiffness tester AB Lorentzen & Wettre at 38°C that is of at least 20% more than the flexibility of said article when measured at 23°C.

2. An article according to claim 1, wherein said article has a flexibility when measured according to stiffness tester AB Lorentzen & Wettre at 38°C that is of 25% more than the flexibility of said article when measured at 23°C, preferably 30% more, more preferably 32% more to 50% more.

3. An article according to any of the preceding claims wherein said article has a flexibility of 100 mN to 10 mN when measured according to stiffness tester AB Lorentzen & Wettre at 23°C, preferably of 80 to 30 mN and more preferably of 60 to 40 mN.

4. An article according to any of the preceding claims wherein said flexibility when measured according to stiffness tester AB Lorentzen & Wettre at 38°C is in the range of 100 mN to 10 mN, preferably of 70 to 15 mN and more preferably of 45 to 20 mN.

5. An article according to any of the preceding claims wherein said article has a total liquid absorption capacity of more than 1 g, as measured on the entire article, more preferably from 2 to 80 g and most preferably from 4 to 40 g.

6. An article according to any of the preceding claims wherein the maximum thickness of said article is less than 3 mm, preferably in the range of 0.5 mm to 1.5 mm, more preferably of 0.7 mm to 1.2 mm and most preferably around 1.0 mm.

7. An article according to any of the preceding claims wherein the article has a flexibility in transverse direction which is at least 10% more than its flexibility in longitudinal direction when measured with a AB Lorentzen & Wettre apparatus at 23°C, preferably at least 40% more and more preferably at least 60% more.

8. An article according to any of the preceding claims, wherein said article comprises a liquid absorbent thermoplastic composition comprising a thermoplastic polymeric base material having particles of water-insoluble water swellable absorbent material dispersed therein, preferably said composition is configured in a plurality of unattached spaced apart zones.

9. An absorbent article according to claim 8 wherein said liquid absorbent thermoplastic composition comprises from 5% to 99% by weight of a polymeric base material comprising a thermoplastic polymer or a mixture thereof, and from 1% to 95% by weight of particles of water insoluble water swellable absorbent material.

10. An absorbent article according to claim 9 wherein said polymeric base material is a hot melt adhesive typically comprising from 10% to 50% by weight of a block copolymer, from 0% to 50% by weight of a tackifying resin, from 10% to 80% by weight of a plasticizer and from 0% to 2.0% by weight of antioxidant.

11. An article according to any of preceding claims 8 to 10, wherein said liquid absorbent thermoplastic composition has a tensile strength in wet state which is at least 20%, preferably at least 40%, and more preferably at least 60%, of the tensile strength of said composition in dry state, said tensile strengths evaluated according to the Tensile Strength Test described herein.

12. An article according to any of the preceding claims 8 to 11, wherein said particles of water-insoluble water swellable absorbent material have a substantially angle-lacking shape and most preferably have an average particle diameter in dry state of below 150 µm.

**13.** An article according to any of claims 8 to 12, wherein said article comprises a liquid pervious surface typically provided per a topsheet, a liquid impervious surface typically provided per a backsheet and an absorbent element sandwiched between the liquid pervious surface and the liquid impervious surface, said absorbent element comprising said liquid absorbent thermoplastic composition comprising a thermoplastic polymeric base material having particles of water-insoluble water swellable absorbent material dispersed therein, typically configured in unattached spaced apart zones, most preferably stripes.

**14.** An article according to claim 13, wherein within the absorbent element the liquid absorbent thermoplastic composition represents at least 15% by weight of the total weight of the absorbent element.

**15.** An article according to any of the preceding claims wherein said article is a feminine protection article, preferably a panty liner or sanitary napkin.

Figure 1

Figure 2

Figure 3

Figure 4

**European Patent Office**

## PARTIAL EUROPEAN SEARCH REPORT

Application Number

which under Rule 45 of the European Patent Convention EP 02 02 1369
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | WO 99 57201 A (FULLER H B LICENSING FINANC ;AHMED SHARF U (US); CLAPP LESLIE J (U) 11 November 1999 (1999-11-11) * page 5, line 6 - line 17 * * page 6, line 1 - line 28 * * page 7, line 10 - line 23 * * page 8, line 7 - line 26 * * page 9, line 11 - page 17 * * page 11, line 15 - line 22 * * page 12, line 6 - line 29 * * page 13, line 16 - line 22 * * page 16, line 23 - page 17, line 2 * * page 21, line 10 - line 16 * * page 21, line 29 - page 22, line 2 * --- | 5,6, 8-10, 12-15 | A61F13/15 A61L15/22 A61L15/60 |
| X,D | EP 1 013 291 A (MCNEIL PPC INC) 28 June 2000 (2000-06-28) * page 2, line 40 - line 49 * * page 3, line 40 - line 53 * * page 4, line 19 - page 5, line 32; examples; table * ----- | 5,6, 8-10, 12-15 | |

**TECHNICAL FIELDS SEARCHED** (Int.Cl.7)

A61F
A61L

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 27 January 2003 | Douskas, K |

EPO FORM 1503 03 82 (P04C07)

**EP 1 402 861 A1**

European Patent Office

**INCOMPLETE SEARCH
SHEET C**

Application Number

EP 02 02 1369

Claim(s) searched completely:
      5, 6, 8-10, 12-15

Claim(s) not searched:
      1-4, 7,11

Reason for the limitation of the search:

Present claims 1-7  relate to an absorbent article and claim 11 to a composition defined by reference to a desirable characteristic or property, namely:

Claims 1-7 :
absorbent article having a flexibility at 38 C that is at least 20% more than the flexibility at 23 C.

Claim 11    :
a thermoplastic composition having a tensile strength in wet state which is at least 20% of the tensile strength in dry state.


The claims cover all products or compositions having this characteristic or property, whereas the application provides support within the meaning of Article 84 EPC and disclosure within the meaning of Article 83 EPC for only a very limited number of such products or compositions. In the present case, the claims so lack support, and the application so lacks disclosure, that a meaningful search over the whole of the claimed scope is impossible. Independent of the above reasoning, the claims also lack clarity (Article 84 EPC). An attempt is made to define the product or composition by reference to a result to be achieved. Again, this lack of clarity in the present case is such as to render a  meaningful search over the whole of the claimed scope impossible. Consequently, the search has been carried out for those parts of the claims which appear to be clear, supported and disclosed, namely those parts relating to the product described in the description the examples and claims 5, 6, 8-10, 12-15.

## ANNEX TO THE EUROPEAN SEARCH REPORT
## ON EUROPEAN PATENT APPLICATION NO.

EP 02 02 1369

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2003

| Patent document cited in search report | | | Publication date | Patent family member(s) | | Publication date |
|---|---|---|---|---|---|---|
| WO 9957201 | A | | 11-11-1999 | AU | 3891899 A | 23-11-1999 |
| | | | | BR | 9910261 A | 02-01-2001 |
| | | | | CN | 1308654 T | 15-08-2001 |
| | | | | EP | 1084194 A1 | 21-03-2001 |
| | | | | JP | 2002526560 T | 20-08-2002 |
| | | | | WO | 9957201 A1 | 11-11-1999 |
| | | | | US | 6458877 B1 | 01-10-2002 |
| EP 1013291 | A | | 28-06-2000 | AU | 6441799 A | 22-06-2000 |
| | | | | BR | 9907422 A | 03-10-2000 |
| | | | | CN | 1261090 A | 26-07-2000 |
| | | | | EP | 1013291 A1 | 28-06-2000 |
| | | | | JP | 2000204333 A | 25-07-2000 |